# EUROPÄISCHE PATENTSCHRIFT

(11) **EP 2 285 329 B1**
(45) Veröffentlichungstag und Bekanntmachung des Hinweises auf die Patenterteilung: **29.05.2013**
(21) Anmeldenummer: 09734905.4
(22) Anmeldetag: 26.03.2009
(51) Int. Cl.: A61F 13/20

(54) **TAMPON**
TAMPON
TAMPON

(30) Priorität: 24.04.2008 DE 102008020640
(43) Veröffentlichungstag der Anmeldung: 23.02.2011
(73) Patentinhaber: Ruhlmann, Frank, 30175 Hannover (DE)
(72) Erfinder: Ruhlmann, Frank, 30175 Hannover (DE)
(74) Vertreter: Wagner, Carsten
(86) Internationale Anmeldenummer: PCT/EP2009/002222
(87) Internationale Veröffentlichungsnummer: WO 2009/129910

(56) Entgegenhaltungen:
- EP-A- 1 310 226
- EP-B- 1 383 453
- WO-A-2008/135925
- US-A- 5 403 300

## Beschreibung

Die Erfindung betrifft einen Tampon der im Oberbegriff des Anspruchs 1 genannten Art.

Derartige Tampons sind allgemein bekannt, beispielsweise durch DE 197 47 633 A1, C1, DE 43 04 305 A1, EP 0 639 363 A1, WO 00 53 141 A, EP 0 597 446 A1, GB 811 756, US 4 328 804, US 5 813 102, DE C 197 47 633, US 3 011 495 und US 4 707 318, und finden in der Frauenhygiene Verwendung.

Durch EP 1 383 453 (entsprechend DE 101 14 786 C2) ist ein Tampon der betreffenden Art bekannt, der einen länglichen Körper mit wenigstens einer ersten Oberflächenrille und wenigstens einer zweiten Oberflächenrille aufweist. Bei dem bekannten Tampon verlaufen die Oberflächenrillen parallel und in Umfangsrichtung des Tampons voneinander beabstandet von einem distalen Einführende in Richtung auf ein proximales Rückholende des Tampons. Sie sind in einer aus geringer verdichtetem Fasermaterial bestehenden Ummantelung des Tampons gebildet und führen in Radialrichtung des Tampons zu einem aus höher verdichtetem Fasermaterial bestehenden Kern. Die Funktion der Oberflächenrillen besteht darin, aufzunehmende Flüssigkeit als Leitkanäle in Axialrichtung des Tampons in Richtung auf das proximale Ende des Tampons zu leiten, wobei die Flüssigkeit in Kontakt mit dem Kern gelangt und von diesem aufgenommen wird. Um die Fläche, auf der die Flüssigkeit in Kontakt mit dem Kern gelangt, zu vergrößern, verlaufen die Oberflächenrillen spiralig um den Körper des Tampons.

Ein ähnlicher Tampon ist auch durch EP 1 459 720 B1 bekannt.

In DE 102 44 874 B4 wird ein Tampon offenbart, mit einem verjüngten Einführende, einem mit einem Rückholmittel versehenen Rückholende einer Längsachse, umfassend einen sich längs erstreckenden Absorptionskörper aus gepresstem Fasermaterial, der im Bereich der Längsachse stärker verdichtet ist und eine Fasersäule bildet, von der sich Längsrippen flankieren, wobei das verjüngte Einführende, das von der Fasersäule, den Längsrillen und Längsrippen gebildet ist, mit Fangrillen und Fangrippen zum Auffangen menstrualer Absonderungen versehen ist, wobei die Fangrillen axial nach vorne und radial nach außen offen sind und die Fangrippen paarweise die Fangrillen flankieren. Die Längsrillen bzw. Längsrippen berühren sich im Scheitelpunkt des Tampons.

In G 94 10 595 U1 ist ein Tampon mit einem länglichen Körper bekannt, der sich in Axialrichtung des Körpers erstreckende Oberflächenrillen aufweist, die, in Umfangsrichtung zueinander beabstandet sind. Um das Flüssigkeitsaufnahmevermögen zu verbessern, weist der Tampon in Axialrichtung zueinander beabstandete Barrierestreifen auf..

Durch US 2004/0199137 A1 ist ein Tampon mit einem länglichen Körper bekannt, der auf seiner Außenfläche mit erhabenen Bändern oder Streifen versehen ist, von denen sich einige in Axialrichtung und andere in umfangsrichtung des Körpers erstrecken. Die Bänder oder Streifen dienen dazu, ein Einführen des Tampons zu erleichtern sowie seine Ausdehnung bei Aufnahme von Flüssigkeit zu begrenzen.

Durch US 5 374 258 ist ein Tampon mit einem länglichen Körper bekannt, der an der Außenfläche mit sich in Axialrichtung erstreckenden Rippen versehen ist, die durch sich in Umfangsrichtung erstreckende faserartige Bänder miteinander verbunden sind.

Die Erfindung liegt die Aufgabe zugrunde, einen Tampon anzugeben, bei dem die Fähigkeit zur Aufnahme von Flüssigkeit verbessert ist.

Diese Aufgabe wird durch die in Anspruch 1 angegebene Lehre gelöst.

Der Grundgedanke der erfindungsgemäßen Lehre besteht darin, die Oberflächenrillen so zu gestalten, daß sich die wenistens eine erste Oberflächenrille und die wenigstens eine zweite Oberflächenrille in ihrem Verlauf berühren. Auf diese Weise kommunizieren die durch die oberflächenrillen gebildeten Leitkanäle für die aufzunehmende Flüssigkeit miteinander, so daß die Aufnahme der Flüssigkeit durch den Tampon in dessen Umfangsrichtung gleichmäßiger gestaltet und dadurch wesentlich verbessert ist.

Unter einer Oberflächenrille im sinne der Erfindung wird jede kanalartige Ausnehmung in der Oberfläche des Tampons verstanden, unabhänigig von ihrem radialen Querschnitt und unabhängig davon, wie sie in dem Körper des Tampons gebildet wird.

Unter einer zur radialen Außenfläche des Körpers offenen Oberflächenrille wird eine Oberflächenrille verstanden, die nicht abgedeckt ist, beispielsweise durch einen Steg oder dergleichen, und damit für einen Eintritt von Flüssigkeit offen ist, unabhängig davon, ob sich gegenüberliegende Wandungen, die die Oberflächenrille begrenzen, im Bereich der radialen Außenfläche des Tampons oder entfernt davon berühren.

Weiterhin erfindungsgemäß verläuft die wenigstens eine erste Oberflächenrille im wesentlichen in Längsrichtung (Axialrichtung) des Körpers des Tampons und die wenigstens eine zweite Oberflächenrille im wesentlichen in Umfangsrichtung des Körpers des Tampons.

Erfindungsgemäß kreuzen sich die wenigstens eine erste und die wenigstens eine zweite Oberflächenrille in ihrem Verlauf. Auf diese Weise ist an der Oberfläche des Tampons insbesondere ein System kommunizierender Leitkanäle geschaffen, durch das einerseits die Aufnahme von Flüssigkeit in Umfangsrichtung des Tampons noch gleichmäßiger gestaltet ist und andererseits die Fläche, auf der die Flüssigkeit in Kontakt mit dem Kern gelangt, wesentlich vergrößert ist. Dadurch ist die Fähigkeit des erfindungsgemäßen Tampons, Flüssigkeit aufzunehmen noch weiter verbessert, so daß für eine Benutzerin des Tampons Komfort und Sicherheit erhöht sind.

Weiterhin erfindungsgemäß verlaufen die wenigstens eine erste Oberflächenrille im wesentlichen in Längsrichtung (Axialrichtung) des Körpers des Tampons und die wenigstens eine zweite Oberflächenriile im wesentlichen in Umfangsrichtung des Körpers des Tampons.

Im Ergebnis ist die Fähigkeit des erfindungsgemäßen Tampons, Flüssigkeit aufzunehmen, gegenüber den bekannten Tampons wesentlich verbessert. Dies gilt auch und insbesondere beim Auftreten von Schwallblutungen, bei denen in relativ kurzer zeit eine relativ große Flüssigkeitsmenge von dem Tampon aufzunehmen ist. Aufgrund der gleichmäßigen Verteilung der Flüssigkeit bietet der erfindungsgemäße Tampon auch bei Schwallblutungen eine erhöhte Sicherheit.

Die Art und Weise, wie die Oberflächenrillen während der Herstellung des Tampons gebildet werden, ist dem Fachmann allgemein bekannt, beispielsweise durch EP 1 383 453 und DE 101 14 786 C2, und wird daher nicht näher erläutert. Mit entsprechenden Werkzeugen lassen sich in dem Körper des Tampons Oberflächenrillen und damit Zeitkanäle mit nahezu beliebigem verlauf und Querschnitt bilden.

Ein weiterer Vorteil des erfindungsgemaßen Tampons besteht darin, daß durch das vorgesehene System an insbesondere eingepreßten Oberflächenrillen ein übermäßiges Aufquellen des Tampons bei gleichzeitig erhöhter Fähigkeit zur Aufnahme von Flüssigkeit vermieden ist. Auf diese Weise ist der Tragekomfort erhöht.

Die Anzahl der ersten und zweiten oberflächenrillen ist erfindungsgemäß entsprechend den jeweiligen Anforderungen innerhalb weiter Grenzen wählbar. Auch der verlauf und der radiale Querschnitt der Oberflächenrillen sind erfindungsgemäß entsprechend den jeweiligen Anforderungen innerhalb weiter Grenzen wählbar.

Der Tampon besteht vorteilhafterweise aus Zellstoff (Cellulose) und/oder aus Polyvinylacetat.

Eine vorteilhafte weiterbildung der Erfindung sieht vor, daß wenigstens eine erste Oberflächenrille von wenigstens einer zweiten Oberflächenrille oder wenigstens eine zweite Oberflächenrille von wenigstens einer ersten Oberflächenrille abzweigt. Bei dieser Ausführungsform bildet die abzweigende Oberflächenrille einen Zweigkanal für die aufzunehmende Flüssigkeit zu der Oberflächenrille, von der sie abzweigt.

Weiterhin erfindungsgemäß weist der erfindungsgemäße Tampon wenigstens zwei erste Oberflächenrillen und vorteilhafterweise wenigstens zwei zweite Oberflächenrillen auf.

Die ersten Oberflächenrillen können sich vorzugsweise von einem distalen Einführende in Richtung auf ein proximales Rückholende des Tampons erstrecken, beispielsweise im wesentlichen in Axialrichtung des Körpers des Tampons. Hierbei kann der Abstand der ersten Oberflächenrillen voneinander in umfangsrichtung des Tampons grundsätzlich unterschiedlich sein. Um die Flüssigkeitsaufnahme in Umfangsrichtung des Tampons besonders gleichmäßig zu gestalten, sieht eine andere Weiterbildung der erfindungsgemäßen Lehre vor, daß die ersten Oberflächenrillen in umfangsrichtung des Tampons im wesentlichen gleich voneinander beabstandet sind.

Eine andere vorteilhafte Weiterbildung der Erfindung sieht vor, daß wenigstens eine erste Oberflächenrille einen im wesentlichen geraden Verlauf und/oder wenigstens eine zweite Oberflächenrille einen im wesentlichen geraden Verlauf aufweist.

Grundsätzlich kann die Rillentiefe, also die Ausdehnung der Oberflächenrillen in Radialrichtung des Körpers des Tampons, bei sämtlichen Oberflächenrillen gleich sein. Um die Flüssigkeitsaufnahme entlang des Tampons gezielt zu beeinflussen, sieht eine andere vorteilhafte Weiterbildung vor, daß die Rillentiefe der Oberflächenrillen zumindest teilweise unterschiedlich ist.

Gemäß einer anderen vorteilhaften Weiterbildung ist die Oberfläche des Körpers durch die Oberflächenrillen in Kompartimente unterteilt. Hierbei können die Oberflächenrillen die Oberfläche des Körpers des Tampons insbesondere rasterartig überziehen, wobei die Kompartimente zwischen den Oberflächenrillen gebildet sind.

Zweckmäßigerweise weist der Körper des Tampons einen Kern aus höher verdichtetem Fasermaterial und eine Ummantelung aus niedriger verdichtetem Fasermaterial auf, in der die Oberflächenrillen gebildet sind. Hierbei dient der im Vergleich zur der Ummantelung stärker saugfähige Kern vorrangig zur Aufnahme der Flüssigkeit, während die Ummantelung dazu dient, die Oberflächenrillen zu definieren.

Eine andere Weiterbildung der Erfindung sieht vor, daß wenigstens eine zweite Oberflächenrille zu wenigstens einer ersten Oberflächenrille orthogonal oder im wesentlichen orthogonal verläuft. Bei dieser Ausführungsform ist es insbesondere möglich, daß die ersten Oberflächenrillen in Axialrichtung des Tampons verlaufen, während die zweiten Oberflächenrillen in Umfangsrichtung des Tampons verlaufen. Es ist erfindungsgemäß jedoch auch möglich, daß die ersten Oberflächenrillen und damit auch die zu den ersten Oberflächenrillen orthogonal oder im wesentlichen orthogonal verlaufenden zweiten Oberflächenrillen zur Längsachse des Tampons geneigt verlaufen.

Eine vorteilhafte Weiterbildung der Ausführungsform mit den Oberflächenrillen unterschiedlicher Tiefe sieht vor, daß wenigstens eine zweite Oberflächenrille eine größere radiale Tiefe als wenigstens eine erste Oberflächenrille aufweist. verlaufen beispielsweise und insbesondere die ersten Oberflächenrillen in Axialrichtung, während die zweiten Oberflächenrillen in umfangsrichtung verlaufen, so ist bei dieser Ausführungsform ein Eintritt von Flüssigkeit, die in die ersten Oberflächenrillen sickert, in die zweiten Oberflächenrillen erleichtert. Auf diese weise wird eine gleichmäßige Verteilung der Flüssigkeit sowohl in Umfangsrichtung als auch in Axialrichtung des Tampons gefördert.

Grundsätzlich können die Oberflächenrillen in der radialen Außenfläche des Tampons auf beliebige geeignete Weise gebildet werden. Eine besonders einfache und kostengünstig herstellbare Ausführungsform sieht vor, daß die Oberflächenrillen in die radiale Außenfläche des Tampons eingepreßt sind. Das Einpressen kann bei der Herstellung des Tampons mittels eines Preßwerkzeugs erfolgen, das an seiner radialen Innenfläche mit hervorstehenden Rippen versehen ist, die sich beim Pressen des Tampons in die Oberflächenrillen abbilden.

Die Erfindung wird nachfolgend anhand der beigefügten Zeichnung näher erläutert, in der als schematische Prinzipsskizzen Ausführungsbeispiele eines erfindungsgemäßen Tampons dargestellt sind.

Es zeigt:
- Fig. 1: eine Perspektivansicht eines Ausführungsbeispieles eines erfindungsgemäßen Tampons,
- Fig. 2: einen Radialschnitt durch den Tampon gemäß Fig. 1,
- Fig. 3: einen Axialschnitt durch den Tampon gemäß Fig. 1.

In den Figuren der Zeichnung sind gleiche oder sich entsprechende Elemente mit den gleichen Bezugszeichen versehen.

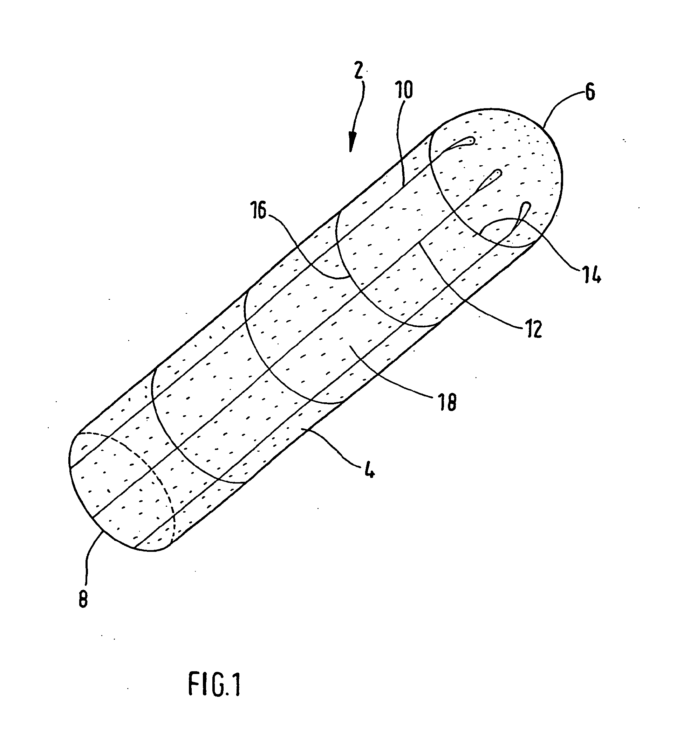

In Fig. 1 ist ein erstes Ausführungsbeispiel eines erfindungsgemäßen Tampons 2 dargestellt, der einen aus einem saugfähigem Material, bei dem dargestellten Ausführungebeispiel einem Fasermaterial, bestehenden; im wesentlichen zylindrischen Körper 4 mit einem distalen Ende 6 (Einführende) und einem proximalen Ende 8 (Rückholende) aufweist. Aus Fig. 1 ist nicht ersichtlich und deshalb wird hier erläutert, daß der Körper 4 einen Kern aus höher verdichtetem Fasermaterial und eine Ummantelung aus geringer verdichtetem Fasermaterial aufweist. Geeignete Materialien für den Kern und die Ummantelung sowei geeignete Herstellungsverfahren für den Tampon 2 sind dem Fachmann allgemein bekannt und werden daher nicht näher erläutert.

Der Tampon 2 weist bei diesem Ausführungsbeispiel eine Mehrzahl von ersten Oberflächenrillen auf, die in Axialrichtung des Körpers 4 verlaufen und in Umfangsrichtung im wesentlichen gleich voneinander beabstandet sind. Fig. 1 sind lediglich zwei der ersten Oberflächenrillen mit den Bezugszeichen 10, 12 versehen. Darüber hinaus weist der Tampon bei diesem Ausführungsbeispiel eine Mehrzahl von zweiten Oberflächenrillen auf, die in Umfangsrichtung des Körpers 4 verlaufen und von denen in Fig. 1 lediglich zwei Oberflächenrillen mit den Bezugszeichen 14, 16 versehen sind. Bei dem dargestellten Ausführungsbeispiel sind die Oberflächenrillen 10, 12, 14, 16 in die radiale Außenfläche des Körpers 2 eingepreßt und bilden somit Preß-Oberflächenrillen.

Erfindungsgemäß berühren sich die ersten Oberflächenrillen 10, 12 und die zweiten Oberlfächenrillen 14, 16 in ihrem Verlauf, was bei dem Ausführungsbeispiel gemäß Fig. 1 dadurch realisiert ist, daß sich die ersten Oberflächenrillen 10, 12 und die zweiten Oberflächenrillen 14, 16 in ihrem Verlauf kreuzen. Wie aus Fig. 1 ersichtlich ist, überziehen die Oberflächenrillen 10, 12, 14, 16 den Körper 4 des Tampons 2 rasterartig, wobei zwischen den Oberflächenrillen 10, 12, 14, 16 Kompartimente gebildet sind, von denen in Fig. 1 lediglich eines mit dem Bezugszeichen 18 versehen ist.

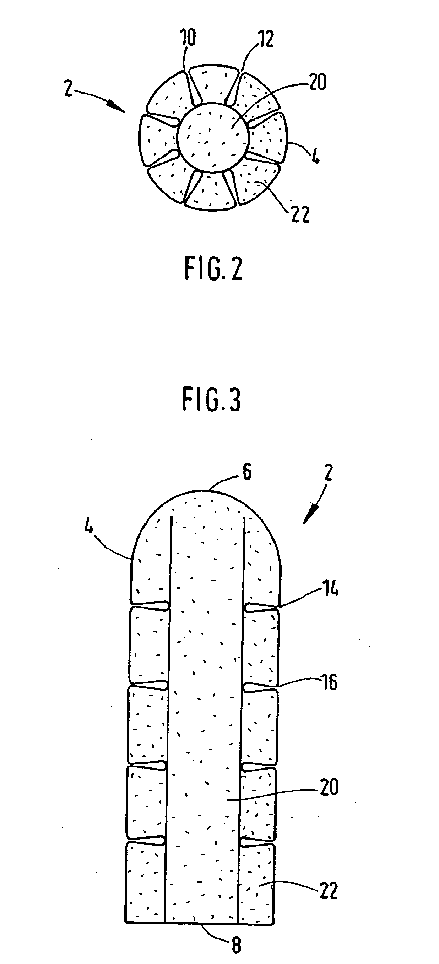

In Fig. 2., die einen Radialschnitt des Tampons 2 zeigt, und Fig. 3 die einen Axialschnitt des Tampons 2 zeigt, sind der Kern 20 und die Ummantelung 22 des Tampons 2 erkennbar. Es ist ersichtlich, daß sich die Oberflächenrillen 10, 12, 14, 16 in Radialrichtung des Körpers 4 von dessen Außenfläche bis zu dem Kern 20 erstrecken. Aus den Fig. 2 und 3 ist ersichtlich, daß die Oberflächenrillen 10, 12, 14, 16 zur radialen Außenfläche des Körpers 4 des Tampons 2 hin offen sind für einen Eintritt von Flüssigkeit, wobei sich die Oberflächenrillen 10, 12, 14, 16 begrenzende Wandungen insbesondere im Bereich der radialen Außenfläche des Körpers 4 und insbesondere im unbenutzten Zustand des Tampons 2 berühren.

Bei Benutzung des Tampons 2 bilden die ersten Oberflächenrillen 10, 12 Leitkanäle, die aufzunehmende Flüssigkeit auf ihrem Fließ- oder Sickerweg von dem distalen Ende 6 des Körpers zu seinem proximalen Ende 8 zu dem Kern 20 leiten, von dem sie aufgenommen wird. Erfindungsgemäß fließt oder sickert die Flüssigkeit in die zweiten Oberflächenrillen 14, 16 ein und wird ach von diesen zu dem Kern 20 geleitet, wobei die zweiten Oberflächenrillen 14, 16 in dem Fließ- bzw. Sickerweg der Flüssigkeit gewissermaßen Zweigkanäle zu den durch die ersten Oberflächenrillen 10, 12 gebildeten Leitkanälen bilden. Auf diese Weise ist die Fläche, auf der die Flüssigkeit in Kontakt mit dem Kern 20 gelangt, wesentlich vergrößert und damit die Aufnahme der Flüssigkeit durch den Kern 20 wesentlich verbessert. Erfindungsgemäß ist damit in der Oberfläche des Körpers 4 ein System miteinander kommunizerender Kanäle gebildet, die die Flüssigkeit sowohl in Axailrichtung als Umfangsrichtung des Körpers 4 gleichmäßig verteilt zu dem Kern 20 leiten. Im Ergebnis sind damit Komfort und Sicherheit für eine Benutzerin des Tampons erhöht.

Der radiale Querschnitt der durch die Oberflächenrillen 10, 12, 14, 16 gebildeten Kanäle ist entsprechend den jeweiligen Anforderungen innerhalb weiter Grenzen wählbar.

## Patentansprüche

1. Tampon, mit einem länglichen Körper, mit wenigstens einer ersten Oberflächenrille und wenigstens einer zweiten Oberflächenrille, die zur radialen Außenfläche des Körpers offen sind, wobei sich die wenigstens eine erste Oberflächenrille und die wenigstens eine zweite Oberflächenrille in ihrem Verlauf zwischen einem proximalen Ende und einem distalen Ende des Körpers kreuzen, wobei die wenigstens eine erste Oberflächenrille im wesentlichen in Axialrichtung des Körpers verläuft und die wenigstens eine zweite Oberflächenrille im wesentlichen in Umfangsrichtung des Körpers verläuft, **dadurch gekennzeichnet, daß** der Tampon wenigstens zwei erste Oberflächenrillen (10, 12) aufweist.

2. Tampon nach Anspruch 1, **dadurch gekennzeichnet, daß** wenigstens eine erste Oberflächenrille (10, 12) von wenigstens einer zweiten Oberflächenrille (14, 16) oder wenigstens eine zweite Oberflächenrille (14, 16) von wenigstens einer ersten Oberflächenrille (10, 12) abzweigt.

3. Tampon nach einem der Ansprüche 1 bis 2, **dadurch gekennzeichnet, daß** dieser wenigstens zwei zweite Oberflächenrillen (14, 16) aufweist.

4. Tampon nach einem der Ansprüche 1 bis 3, **dadurch gekennzeichnet, daß** die ersten Oberflächenrillen (10, 12) in Umfangsrichtung im wesentlichen gleich voneinander beabstandet sind.

5. Tampon nach einem der Ansprüche 1 bis 4, **dadurch gekennzeichnet, daß** die wenigstens eine erste Oberflächenrille (10, 12) einen im wesentlichen geraden Verlauf und/oder die wenigstens eine zweite Oberflächenrille (14, 16) einen im wesentlichen geraden Verlauf aufweist.

6. Tampon nach einem der Ansprüche 1 bis 5, **dadurch gekennzeichnet, daß** die Rillentiefe der Oberflächenrillen (10, 12, 14, 16) zumindest teilweise unterschiedlich ist.

7. Tampon nach einem der Ansprüche 1 bis 6, **dadurch gekennzeichnet, daß** die Oberfläche des Körpers durch die Oberflächenrillen (10, 12, 14, 16) in Kompartimente unterteilt ist.

8. Tampon nach einem der Ansprüche 1 bis 7, **dadurch gekennzeichnet, daß** der Körper einen Kern aus höher verdichtetem Fasermaterial und eine Ummantelung aus niedriger verdichtetem Fasermaterial aufweist, in der die Oberflächenrillen gebildet sind.

9. Tampon nach einem der Ansprüche 1 bis 8, **dadurch gekennzeichnet, daß** die wenigstens eine erste Oberflächenrille (10, 12) und/oder die wenigstens eine zweite Oberflächenrille (14, 16) wellenförmig verläuft bzw. verlaufen.

10. Tampon, nach einem der vorhergehenden Ansprüche, **dadurch gekennzeichnet, daß** wenigstens eine zweite Oberflächenrille (14, 16) zu wenigstens einer ersten Oberflächenrille (10, 12) orthogonal oder im wesentlichen orthogonal verläuft.

11. Tampon, nach einem der vorhergehenden Ansprüche, **dadurch gekennzeichnet, daß** wenigstens eine zweite Oberflächenrille (14, 16) eine größere radiale Tiefe als wenigstens eine erste Oberflächenrille (10, 12) aufweist.

12. Tampon, nach einem der vorhergehenden Ansprüche, **dadurch gekennzeichnet, daß** die Oberflächenrillen (10, 12, 14, 16) in die radiale Außenfläche des Tampons (2) eingepresst sind.

## Claims

1. Tampon with an elongated body, having at least one first surface groove and at least one second surface groove, which are open towards the radial outer surface of the body, wherein the at least one first surface groove and the at least one second surface groove cross in their path between a proximal end and a distal end of the body, wherein the at least one first surface groove runs essentially in axial direction of the body and the at least one second surface groove runs essentially in circumferential direction of the body, **characterised in that** the tampon has at least two first surface grooves (10, 12).

2. Tampon according to claim 1, **characterised in that** at least one first surface groove (10, 12) branches off at least one second surface groove (14, 16) or at least one second surface groove (14, 16) of at least one first surface groove (10, 12).

3. Tampon according to any one of claims 1 to 2, **characterised in that** the latter comprises at least two second surface grooves (14, 16).

4. Tampon according to any one of claims 1 to 3, **characterised in that** the first surface grooves (10, 12) are spaced essentially evenly apart from one another in circumferential direction.

5. Tampon according to any one of claims 1 to 4, **characterised in that** the at least one first surface groove (10, 12) follows an essentially straight path and/or the at least second surface groove (14, 16) follows an essentially straight path.

6. Tampon according to any one of claims 1 to 5, **characterised in that** the groove depth of the surface grooves (10, 12, 14, 16) is at least partly different.

7. Tampon according to any one of claims 1 to 6, **characterised in that** the surface of the body is divided into compartments by the surface grooves (10, 12, 14, 16).

8. Tampon according to any one of claims 1 to 7, **characterised in that** the body has a core made of more compacted fibre material and a casing made of less-compacted fibre material in which the surface grooves are formed.

9. Tampon according to any one of claims 1 to 8, **characterised in that** the at least one first surface groove (10, 12) and/or the at least one surface groove (14, 16) is or are wave-like.

10. Tampon according to any one of the preceding claims, **characterised in that** at least one second surface groove (14, 16) runs orthogonally or essentially orthogonally to at least one first surface groove (10, 12).

11. Tampon according to any one of the preceding claims, **characterised in that** at least one second surface groove (14, 16) has a greater radial depth than at least one first surface groove (10, 12).

12. Tampon according to any one of the preceding claims, **characterised in that** the surface grooves (10, 12, 14, 16) are pressed into the radial external surface of the tampon (2).

## Revendications

1. Tampon hygiénique, comprenant un corps allongé avec au moins une première rainure superficielle et au moins une deuxième rainure superficielle ouvertes vers la face extérieure radiale du corps, dans lequel ladite au moins une première rainure superficielle et ladite au moins une deuxième rainure superficielle se croisent au niveau de leur trajectoire entre une extrémité proximale et une extrémité distale du corps, dans lequel ladite au moins une première rainure superficielle s'étend substantiellement dans la direction axiale du corps et ladite au moins une deuxième rainure superficielle s'étend substantiellement dans la direction circonférentielle du corps, **caractérisé en ce que** le tampon hygiénique présente au moins deux premières rainures superficielles (10, 12).

2. Tampon hygiénique selon la revendication 1, **caractérisé en ce qu'**au moins une première rainure superficielle (10, 12) bifurque de ladite au moins une deuxième rainure superficielle (14, 16) ou **en ce qu'**au moins une deuxième rainure superficielle (14, 16) bifurque de ladite au moins une première rainure superficielle (10, 12).

3. Tampon hygiénique selon l'une quelconque des revendications 1 à 2, **caractérisé en ce qu'**il présente au moins deux deuxièmes rainures superficielles (14, 16).

4. Tampon hygiénique selon l'une quelconque des revendications 1 à 3, **caractérisé en ce que** les premières rainures superficielles (10, 12) sont espacées les unes des autres dans la direction circonférentielle de manière substantiellement identique.

5. Tampon hygiénique selon l'une quelconque des revendications 1 à 4, **caractérisé en ce que** ladite au moins une première rainure superficielle (10, 12) présente une trajectoire substantiellement droite et/ou **en ce que** ladite au moins une deuxième rainure superficielle (14, 16) présente une trajectoire substantiellement droite.

6. Tampon hygiénique selon l'une quelconque des revendications 1 à 5, **caractérisé en ce que** la profondeur de rainure des rainures superficielles (10, 12, 14, 16) est différente au moins en partie.

7. Tampon hygiénique selon l'une quelconque des revendications 1 à 6, **caractérisé en ce que** la surface du corps est divisée en compartiments par les rainures superficielles (10, 12, 14, 16).

8. Tampon hygiénique selon l'une quelconque des revendications 1 à 7, **caractérisé en ce que** le corps présente un noyau en matériau fibreux de plus grande densité et une gaine en matériau fibreux de moindre densité et dans laquelle sont formées les rainures superficielles.

9. Tampon hygiénique selon l'une quelconque des revendications 1 à 8, **caractérisé en ce que** ladite au moins une première rainure superficielle (10, 12) et/ou ladite au moins une deuxième rainure superficielle (14, 16) s'étend(ent) de manière ondulée.

10. Tampon hygiénique selon l'une quelconque des revendications précédentes, **caractérisé en ce qu'**au moins une deuxième rainure superficielle (14, 16) s'étend de manière orthogonale ou substantiellement orthogonale à au moins une première rainure superficielle (10, 12).

11. Tampon hygiénique selon l'une quelconque des revendications précédentes, **caractérisé en ce qu'**au moins une deuxième rainure superficielle (14, 16) présente une plus grande profondeur radiale qu'au moins une première rainure superficielle (10, 12).

12. Tampon hygiénique selon l'une quelconque des revendications précédentes, **caractérisé en ce que** les rainures superficielles (10, 12, 14, 16) sont gaufrées dans la face extérieure radiale du tampon (2).
